Europäisches Patentamt

European Patent Office

Office européen des brevets

(11) Publication number: **0 071 598**
**B1**

(12) **EUROPEAN PATENT SPECIFICATION**

(45) Date of publication of patent specification: **09.05.90**

(51) Int. Cl.⁵: **A 61 K 7/06**, A 61 K 31/215

(21) Application number: **81901199.0**

(22) Date of filing: **30.03.81**

(86) International application number:
**PCT/US81/00338**

(87) International publication number:
**WO 82/02833 02.09.82 Gazette 82/21**

(54) **THE USE OF RETINOIDS AND THEIR DERIVATIVES TO INCREASE THE RATE OF GROWTH OF HUMAN SCALP HAIR AND TO INCREASE THE RATE OF GROWTH OF FUR IN CERTAIN FURBEARING ANIMALS.**

(30) Priority: **17.02.81 US 235169**

(43) Date of publication of application:
**16.02.83 Bulletin 83/07**

(45) Publication of the grant of the patent:
**09.05.90 Bulletin 90/19**

(84) Designated Contracting States:
**AT CH DE FR GB LI LU NL SE**

(73) Proprietor: **BAZZANO, Gail S**
**4506 Avron Blvd.**
**Metairie, LA 70002 (US)**

(72) Inventor: **BAZZANO, Gail S**
**4506 Avron Blvd.**
**Metairie, LA 70002 (US)**

(74) Representative: **Casalonga, Axel et al**
**BUREAU D.A. CASALONGA - JOSSE**
**Morassistrasse 8**
**D-8000 München 5 (DE)**

(56) References cited:
EP-A-0 010 208   US-A-3 931 279
FR-A-1 297 730   US-A-3 954 835
FR-A-2 247 203   US-A-4 034 114
FR-A-2 293 193   US-A-4 194 007
GB-A-1 466 062   US-A-4 214 000
US-A-3 759 978

British J. of Dermatology (1984) 110 p. 685-689
Revue de la Médecine No 36, (10) 1980 p. 1855-1858

The file contains technical information submitted after the application was filed and not included in this specification

(56) References cited:
Recherche (1985) No 2
J.O.C.E. (270976) No 1262 p. 169
Code de la Santé Publique (Annexe 5)
Règlementation des medicaments dans la CEE
EXPERIENTIA, vol.33, Fasc. 8, August 15,1977, E.S. WILKINS et al.: "Increased relative growth rate of normal rat cells in vitro with crocetin", page 1028

CHEMICAL ABSTRACTS, vol. 84, no.21, May 24,1976, page 461, abstract no. 149559f, COLUMBUS, OHIO (US) & JP A 75 161 465
Food and Drug (F.D.C.A) 21 paragraph 321
Medical Times (5) 1963, p. 385-387

Courier Press, Leamington Spa, England.

EP 0 071 598 B1

**Description**

This invention relates to the use of pharmaceutical or veterinary compositions containing retinoids and their derivatives for treating alopecia by increasing the rate of growth of hair and prolonging the anagen phase of the hair cycle in mammals and to increase the rate of growth of fur in certain fur bearing animals.

Many factors may influence the rate of hair growth including race, sex, age, region, season of the year, nutrition and hormones (Myers and Hamilton, 1951, Hamilton, 1958, Yano, 1936, Maeda, 1938, Troter, 1923, Pinkus, 1924, and Ono, 1963). In the past, many attempts have been made to alter the course of male pattern alopecia through numerous types of treatments, all of which have proven unsuccessful. This invention describes a treatment of alopecia by increasing the rate of growth of hair from growing hair follicles and from the hair follicles of certain fur bearing animals, and by prolonging the growing phase or anagen phase of the hair cycle.

In the past, Vitamin A compounds hereinafter referred to as retinoids have been shown to be effective in treating many types of disorders including dermatological conditions such as acne and psoriasis.

GB A 1.466.062 describes the use or retinoic acid in cosmetic preparations to be applied to the human skin especially, the face and hands, hair and nails. This document notices that due to the reduction or elimination of spots, pimples, and dandruff the scalp condition is improved and hair loss is reduced or arrested and due to the thickening of the epidermis hair growth is recommenced.

No mention is however made on an eventual effect on the principal biological mechanism of hair growth and on its effect on alopecia.

I have now found a new use for the retinoids in the treatment of alopecia by increasing the rate of hair growth and prolonging the anagen phase of the hair cycle.

I have discovered that retinoids, particularly alltrans-retinoic acid and its derivatives, can treat alopecia by increasing the rate of scalp hair growth in humans and that it can also be used to increase the rate of growth of fur in certain fur bearing animals and to retard moulting.

The choice of administration is topically by use of lotions, ointments and creams. However, administration is not limited to these means, the effect of can also be obtained by the oral or systemic administration of the retinoid compounds.

This invention concerns the use of retinoids, including all-trans-retinoic acid and its metabolites and other retinoid compounds in order to treat alopecia by increasing the rate of scalp hair growth in humans and to increase the rate of pelt (fur) growth in certain fur bearing animals. All-trans-retinoic acid has been shown to cause elevated DNA synthesis in keratinocytes in celle culture. All-trans-retinoic acid can also be shown to increase the turnover time of epidermal cells in cell culture experiments as well as *in vivo* experiments with human subjects. I have discovered that the cells of the hair follicle including the papillae can be stimulated by retinoids including all-trans-retinoic acid. When treated experimentally, the retinoids caused the cells of the dermal papillae and the cells of the root sheath to incorporate more tritiated thymidine into DNA and to reproduce at a more rapid rate than untreated cells from other hair follicles. This stimulation by the retinoid compounds ultimately causes the entire hair follicle to become more activated and the mitotic index is measured by thymidine-$H^3$ incorporation into DNA to rise. Therefore the individual scalp hairs can be shown to grow at an increased rate and the anagen phase is prolonged.

A major problem in influencing hair growth is to obtain good percatenenous absorption of the active compounds.

Another problem is the accurate measurement of hair growth to substantiate the results of the testing. All-trans-retonoic acid and its derivatives and the other retinoid compounds have been shown to give excellent percutaneous absorption and to be very active on the keratinizing cells of the skin, including the hair follicle. Furthermore, a relatively recent method which gives excellent results has been used to measure the rate of hair growth. This method was devised by Saitoh. M. et al and utilises microcapillary tubes which are graduated using 0.2 mm graduations.

As used herein, the term retinoid denotes retinol, retinal, retinyl esters as well as retinoic acid and its esters, derivatives and normal metabolites. The terminal group may be oxidized, reduced or esterified. The alkali metal (sodium or potassium, etc) and alkaline earth metal (magnesium or calcium) salts are also included herein.

The compounds more particularly used according to invention are:

all-trans retinoic acid, all-trans retinaldehyde or all-trans retinoyl acetate or other suitable esters or salts of retinoic acid;

the stereoisomers or vitamin A acid, or Vitamin $A_2$ acid, γ-vitamin A acid, α-vitamin A acid, 5-6-epoxy vitamin A acid, dehydrovitamin A acid or the aldehydes, alcohols, esters, ethers or salts of the above mentioned compounds;

naturally occurring metabolites of vitamin A acid such as 4-hydroxy retinoic acid, 4-keto-retinoic acid, 4-oxo-retinoic acid, 5,8-oxy retinoic acid or 7-cis-3-dehydroetinol or retinoic acid, 7-trans-9-cis-11-trans-13-trans-5'-hydroxy retinoic acid, or 8-(2,6,6-trimethyl-3-oxocyclohex-1-enyl)-2,6 dimethyl-6-hydroxy-octotrienal or stereoisomers of the above mentioned compounds or aldehydes or derivatives such as esters or salts thereof;

carotenoids such as crocetin or crocin or its naturally occurring metabolites.

The pharmaceutical or veterinary preparations of the present invention can be prepared by the

2

conventional techniques for the preparation of lotions, creams, conditioners or shampoos for the scalp or veterinary preparations for pelts. Included also are preparations which can be administered orally and compounds which can be added to animal feeds.

In addition to the active retinoids of this invention, the various preparation can contain any conventional pharmaceutically acceptable inert or pharmacodynamically active additives. For example, the lotions may be prepared using various forms of alcohol or other solubilizers such as glycols, or esters. The conditioners may contain the normally acceptable commercially produced compounds such as cetyl alcohol, ceteareth-5,-20 hydantoins, hydrolyzed animal protein, glycol stearate, amodimethicone, paraffin and mineral oil. (These are only given as examples, and are not meant to be exclusive.) The topical compositions may also contain various oils including essential fatty acids and other polyunsaturated fatty acids and their derivatives, and compounds such as hormones, including progesterone, estradiols and thyroid.

The topically applied lotions, creams, and conditioners, will vary according to the standard art with regard to the amounts of other hydrophilic and hydrophobic containing ingredients including emulsifiers, so that either an oily semi-oily or oil free product may be obtained. The shampoos may contain any of the conventionally used detergents or soaps and any other compounds used by those familiar with the art. Oil based shampoos are included in these formulations.

The oral presentations may be tablets, liquids or capsules. The pharmaceutically acceptable substances commonly used as preservatives, stabilizers, moisture retainers and emulsifiers can be present in these preparations. Conventionally. acceptable antioxidants such as tocopherols, N-methyl a-tocopheramine, butylated hydroxyanisole and butylated hydroxytoluene, can be also incorporated in the preparations described in this invention.

The dosage in which the retinoids are administered can be varied with the route of administration and the requirements of the subjects.

The compositions to be used for topical treatments may consist of lotions, creams, conditioners, shampoos and oil with from 0.001 to 2% by weight of all-trans-retinoic acid or derivatives, or other retinoids, as the preferred dosages in the described compositions.

The oral pharmaceutical preparations may be administered orally or can also be incorporated into animal chow in the case of fur bearing animals, i.e. in the case of dogs and cats, or other pet food or in the case of bird seed for moulting birds.

In order to examine the specific action of the retinoids in treating alopecia by increasing the rate of hair growth, several types of experiments were performed. The microcapillary method was used in each case to measure the rate of hair growth.

In tables I—VI is described the results of studies using male and female subjects. The all-trans-retinoic acid was applied topically or as described in the tables and the microcapillary method was used to assess hair growth rates.

In Table II is described the results of studies using male and female subjects. The ethyl ester of all-trans-retinoic acid was applied topically or as described in the table and a control non-treated area was utilized for comparison.

In fur bearing animals, the rate of fur growth length of hair, thickness of hair and moulting season are controlled by many factors including seasons, light (wavelength), periodicity, temperature, hormonal factors and nutrition. Controlling all these variables is impossible however, animals were selected and areas over the hind quarters were shaved in 5.08 cm (2 inch) diameter circular areas. In some of the animals the areas were treated topically with all-trans-retinoic acid and in other animals the retinoid was administered orally in animal chow. Some of the animals served as their own controls, using treated and non-treated areas.

In fur bearing animals, the guard hairs and the pile hairs differ in thickness, length and growth rate. In the rabbits studied, the guard hairs averaged 34 mm and the pile hairs 30 mm in length. The effect of topical application of all-trans-retinoic acid was to increase the rate of new hair growth. An effect on the non-shaved fur bearing areas treated topically with all-trans-retinoic acid in lotion form, was a decrease in the shedding or moulting of fur. The mean rate of hair (fur) growth from treated shaved areas was 0.3 mm/day for 3 rabbits (mean) while in non-treatment shaved areas it averaged 0.2 mm per day (mean of 3 rabbits).

The effect could also be demonstrated in domestic cats and dogs; the same type of experimental procedures were used. The most striking effect in long haired dogs and cats was the retardation of moulting or hair shedding. Long haired dogs and cats tended to retain more hair in the anagen phase and there was approximately 50% less shedding during the treatment periods. Both methods of administration were satisfactory. Either topical lotion or cream treatment or systemic treatment by inclusion in animal chow was satisfactory.

Commercially important fur bearing animals were also used for experimentation. Two male minks were closely clipped over the back hind quarters. The animals were treated on one hind quarter and the other was used as the control. The capillary method for measuring hair growth was used for these studies. The animals were treated by two different methods. The animals were either fed the retinoid in their chow or they were administered the retinoid topically. The results of these experiments showed that the rate of growth of new pelt was increased approximately 30% by the retinoid treatment.

Experiments using birds (canaries and parakeets) showed that inclusion of the all-trans-retinoic acid or

the ethyl ester of all-trans-retinoic acid in bird food retarded the moulting process.

The following Examples illustrate the present invention. The methods of administration may vary by lotion, cream, ointment, pill, supplement to chow or coating for seeds. These Examples are only meant to be illustrative and do not limit the mode of administration nor the ingredients which can be admixed to the present invention, nor the amounts which may be used.

### Example I
Lotion formulation for the topical administration

|  | % wt to wt |
| --- | --- |
| Active ingredient: all trans retinoic acid | 0.1 |
| Ethanol | q.s. to 100.00 |
| Propylene glycol | 5.0 |
| Butylated hydroxytoluene | 0.1 |
| Safflower oil | 1.0 |
| α-tocopherol acetate | 0.5 |

### Example II
Cream conditioner for Topical Administration

| Active ingredient: All-trans-retinoic acid or all trans-retinoyl acetate | 1.0 |
| --- | --- |
| Distilled water | q.s. to 100.0 |
| Cetrimonium Chloride | 5.0 |
| cetyl alcohol | 4.0 |
| Ethanol | 4.0 |
| Butylated hydroxytoluene | 1.0 |
| hydrolyzed animal protein | 0.5 |
| methylparaben, propylbaren | 0.1 |

### Example III

All-trans-retinoic acid 0.1 gram is dissolved in 10 ml of acetone, and the solution admixed with 90 g of USP grade hydrophilic ointment to a uniform consistency; one gram of butylated hydroxytoluene is added. The water washable cream ointment thus prepared consists of 0.1% active ingredient.

### Example IV
Tablets for oral administration.

| Active ingredient: all-trans-retinoic acid or all-trans-retinoic acid ethyl ester or acetate | 25 mg |
| --- | --- |
| Lactose | 52 mg |
| Cornstarch | 20 mg |
| Microcrystalline cellulose | 40 mg |
| Talc | 2.5 mg |
| Magnesium stearate | 0.5 mg |

The active ingredient was mixed with lactose and granulated using a corn starch paste. The remainder of the above adjuvants was then admixed therein and the mass was tableted. The tablets were then tableted. The tablets were then coated with a water-soluble or water-swellable lacquer.

4

The same formulation can also be used and gelatin can be added to make beadlets. These beadlets are then coated with a lacquer. The beadlets and animal chow can be mixed to the desired dosage level.

The above formulation can also be used in the powdered form for mixing with bird seed and the bird seed can then be sprayed with lacquer.

Liquids, syrups or other formulations can be made consistent with the pharmaceutical art.

TABLE I
Results using all-trans-retinoic acid

| Subject | | | Form of Dosage | Treatment Time | mm/day Control Rate | mm/day Treatment Rate of Growth |
|---|---|---|---|---|---|---|
| Sex | Age | Dosage | | | | |
| M | 36 | 10 mg/day | Topical Lotion | 21 days | 0.3 | 0.45 |
| M | 38 | 60 mg/day | oral | 14 days | 0.21 | 0.25 |
| M | 24 | 10 mg | Topical | 10 days | 0.35 | 0.49 |
| F | 35 | 10 mg | Topical | 30 days | 0.39 | 0.50 |
| F | 41 | 60 mg | Oral | 28 days | 0.36 | 0.39 |
| F | 63 | 10 mg | Topical | 21 days | 0.28 | 0.38 |

TABLE II
Results using all-trans-retinoic acid ethyl ester

| Subject | | | Form of Dosage | Treatment Time | mm/day Control Rate | mm/day Treatment Rate of Growth |
|---|---|---|---|---|---|---|
| Sex | Age | Dosage per day | | | | |
| M | 31 | 10 mg | Topical | 21 days | 0.25 | 0.31 |
| M | 68 | 10 mg | Topical | 20 days | 0.21 | 0.29 |
| M | 38 | 70 mg | Oral | 21 days | 0.30 | 0.32 |
| F | 36 | 10 mg | Topical | 22 days | 0.34 | 0.46 |
| F | 42 | 10 mg | Topical Cream | 19 days | 0.31 | 0.40 |
| F | 66 | 50 mg | Oral | 20 days | 0.28 | 0.29 |

TABLE III
Results using all-trans-retinaldehyde
(Commercially available — for synthesis see Merck Index)

| Subject | | | Form of Dosage | Treatment Time | mm/day Control Rate | mm/day Treatment Rate of Growth |
|---|---|---|---|---|---|---|
| Sex | Age | Dosage | | | | |
| M | 36 | 10 mg/day | Topical | 21 days | 0.30 | 0.35 |
| M | 38 | 10 mg/day | Topical | 14 days | 0.23 | 0.32 |
| M | 24 | 10 mg | Topical | 10 days | 0.35 | 0.40 |
| F | 35 | 10 mg | Topical | 30 days | 0.39 | 0.45 |
| F | 41 | 10 mg | Topical | 28 days | 0.31 | 0.35 |
| F | 63 | 10 mg | Topical | 21 days | 0.28 | 0.41 |

TABLE IV
Results using Retinoic acid metabolites
(i.e. 4-hydroxy and 4-keto)
(Can be prepared by enzymatic methods; synthesis known)
Ref: Archives of Biochemistry and Biophysics, 199 p.374—383 (1980)

| Subject | | Dosage per day | Form of Dosage | Treatment Time | Control Rate | Treatment Rate of Growth |
|---|---|---|---|---|---|---|
| Sex | Age | | | | | |
| M | 31 | 10 mg | Topical | 21 days | 0.25 | 0.31 |
| M | 68 | 10 mg | Topical | 20 days | 0.21 | 0.28 |
| M | 38 | 10 mg | Topical | 21 days | 0.34 | 0.35 |
| F | 36 | 10 mg | Topical | 22 days | 0.34 | 0.41 |
| F | 42 | 10 mg | Topical | 19 days | 0.39 | 0.48 |
| F | 66 | 10 mg | Topical | 20 days | 0.28 | 0.36 |

TABLE V
Results using oxidation products of retinoic acid
i.e 8-(2,6,6-trimethyl-3-oxocyclohex-1-enyl)-2,6-dimethyl-6-hydroxoctatrienal prepared by enzymatic methods (Biochimica Biophysica Acta, 627) 270—275 (1980)

| Subject | | Dosage | Form of Dosage | Treatment Time | mm/day Control Rate | mm/day Treatment Rate of Growth |
|---|---|---|---|---|---|---|
| Sex | Age | | | | | |
| M | 36 | 10 mg/day | Topical | 21 days | 0.30 | 0.35 |
| M | 38 | 10 mg/day | Topical | 14 days | 0.21 | 0.24 |
| M | 24 | 10 mg | Topical | 10 days | 0.35 | 0.35 |
| F | 35 | 10 mg | Topical | 30 days | 0.39 | 0.45 |
| F | 41 | 10 mg | Topical | 28 days | 0.36 | 0.38 |
| F | 63 | 10 mg | Topical | 21 days | 0.28 | 0.27 |

TABLE VI
Results using dehydroretinols extracted from pike liver oil and their stereoisomers (see Merck Index for specifications) and oxidation products including aldehydes and acids

| Subject | | Dosage | Form of Dosage | Treatment Time | mm/day Control Rate | mm/day Treatment Rate of Growth |
|---|---|---|---|---|---|---|
| Sex | Age | | | | | |
| M | 31 | 10 mg | Topical | 21 days | 0.25 | 0.41 |
| M | 68 | 10 mg | Topical | 20 days | 0.21 | 0.25 |
| M | 38 | 10 mg | Topical | 21 days | 0.30 | 0.34 |
| F | 36 | 10 mg | Topical | 22 days | 0.34 | 0.34 |
| F | 42 | 10 mg | Topical | 19 days | 0.31 | 0.32 |
| F | 66 | 10 mg | Topical | 20 days | 0.28 | 0.29 |

# EP 0 071 598 B1

## Claims

1. The use of a retinoid or of a pharmaceutically acceptable ester, ether, or salt thereof, for the manufacture of a pharmaceutical or veterinary composition suitable for topical or oral administration to mammals for treating alopecia by increasing the growth of hair and prolonging the anagen phase of the hair cycle.

2. The use of all-trans-retinoic acid, or all-trans-retinaldehyde, or all-trans-retinoyl acetate, or other suitable esters or salts of retinoic acid for the purpose as defined in claim 1.

3. The use of a retinoid selected from the stereoisomers of vitamin A acid, or vitamin $A_2$ acid, γ-vitamin A acid, α-vitamin A acid, 5,6 epoxy-vitamin A acid, dehydrovitamin A acid or the aldehydes, alcohols, esters, ethers or salts of the above mentioned compounds for the purpose as defined in claim 1.

4. The use of a naturally occurring metabolite of vitamin A acid such as 4-hydroxy-retinoic acid, 4-keto-retinoic acid, 4-oxo-retinoic acid, 5,8-oxy-retinoic acid or 7-cis-3-dehydroretinol or retinoic acid, 7-trans-9-cis-11 trans-13-trans-5'-hydroxy-retinoic- acid, or 8-(2,6,6-trimethyl-3-oxocyclohex-1-enyl)-2,6-dimethyl-6-hydroxy-octatrienal or stereoisomers of the above mentioned compounds, or aldehydes or derivatives such as esters or salts thereof, for the purpose as defined in claim 1.

5. The use of carotenoid such as crocetin or crocin or its naturally occurring metabolites, for the purpose as defined in claim 1.

6. The use of a retinoid as defined in any one of claims 1 to 5 for the manufacture of a pharmaceutical composition suitable for topical administration for treating human alopecia by increasing the rate of hair growth and prolonging the anagen phase of the hair cycle.

7. The use of a retinoid as defined in any one of claims 1 to 5 wherein the mammal is a fur bearing animal and the composition also acts as a remedy against moulting or shedding.

8. The use for the purpose as defined in claims 1 to 6 wherein the pharmaceutical or veterinary composition is formulated as a topical lotion, cream, ointment, conditioner or shampoo and the retinoid concentration is between 0.001 to 5% by weight.

9. Use as defined in claim 8 wherein the topical lotion contains as solubilizers a mixture of ethanol and propylene glycol.

10. Method of treatment of fur bearing animals in order to increase the rate of growth of the fur characterized in that an effective amount of a retinoid as defined in any one of claims 1 to 5 is administered to the animals.

## Patentansprüche

1. Verwendung eines Retinoids oder eines pharmazeutisch verträglichen Esters, Ethers oder Salzes davon zur Herstellung einer pharmazeutischen oder tiermedizinischen Zusammensetzung, die geeignet ist zur topischen oder oralen Verabreichung an Säugetieren zur Behandlung von Alopecia, indem sie die Wachstumsrate des Haares steigert und die Anagenphase des Haarzyklus verlängert.

2. Verwendung von Gesamt-trans-Retinoinsäure oder Gesamt-trans-Retinaldehyd oder Gesamt-trans-Retinoylacetat oder anderer geeigneter Ester oder Salze der Retinoinsäure für den in Anspruch 1 bezeichneten Zweck.

3. Verwendung eines Retinoids, ausgewählt aus den Stereoisomeren von Vitamin A-Säure oder Vitamin $A_2$-Säure, gamma-Vitamin A-Säure alpha-Vitamin A-Säure, 5,6-Epoxy-Vitamin A-Säure, Dehydro-Vitamin A-Säure oder den Aldehyden, Alkoholen, Estern, Ethern oder Salzen der oben genannten Verbindungen, für den in Anspruch 1 bezeichneten Zweck.

4. Verwendung eines natürlich auftretenden Metaboliten von Vitamin A-Säure, wie 4-Hydroxy-retinoinsäure, 4-Keto-retinoinsäure, 4-Oxo-retinoinsäure, 5,8-Oxy-retinoinsäure oder 7-cis-3-Dehydroretinol oder -retinoinsäure, 7-trans-9-cis-11-trans-13-trans-5'-Hydroxy-retinoinsäure, oder 8-(2,6,6-Trimethyl-3-oxycyclohex-1-enyl)-2,6-dimethyl-6-hydroxy-octatrienal oder Stereoisomeren der oben genannten Verbindung, oder Aldehyde oder Derivaten, wie Est er oder Salze davon, für den in Anspruch 1 bezeichneten Zweck.

5. Verwendung eines Karotenoids, wie Crocetin oder Crocin oder natürlich auftretender Metabolite davon für den in Anspruch 1 bezeichneten Zweck.

6. Verwendung eines gemäss einem der Ansprüche 1 bis 5 bezeichneten Retinoids für die Herstellung einer pharmazeutisches Zusammensetzung, die geeignet ist für die topische Verabreichung zur Behandlung von Alopecia beim Menschen, indem sie die Wachstumsrate des Haares steigert und die Anagenphase des Haarzyklus verlängert.

7. Verwendung eines gemäss einem der Ansprüche 1 bis 5 bezeichneten Retinoids, wobei das Säugetier ein felltragendes Tier ist, und die Zusammentsetzung auch als Heilmittel gegen Mauserung wirkt.

8. Verwendung für den in den Ansprüchen 1 bis 6 bezeichneten Zweck, wobei die pharmazeutische oder tiermedizinische Zusammensetzung als eine topische Lotion, Creme, Salbe, Konditionierzubereitung oder als Shampoo formuliert ist, und die Retinoidkonzentration zwischen 0,001 und 5 Gew.% liegt.

9. Verwendung nach Anspruch 8, wobei die topische Lotion als Lösungsungsvermittler eine Mischung von Ethanol und Propylenglykol enthält.

10. Verfahren zur Behandlung von felltragenden Tieren, um die Wachstumsrate des Felles zu steigern, dadurch gekennzeichnet, dass eine wirksame Menge eines gemäss einem der Ansprüche 1 bis 5 bezeichneten Retinoids den Tieren verabreicht wird.

**Revendications**

1. Utilisation d'un rétinoïde ou d'un sel, éther ou ester pharmaceutique acceptable de celui-ci, pour la fabrication d'une composition pharmaceutique ou vétérinaire, appropriée pour une administration topique ou orale à des mammifères pour le traitement de l'alopécie par augmentation de la vitesse de croissance des cheveux et prolongation de la phase anagène du cycle de cheveu.

2. Utilisation de l'acide tout-trans-rétinoïque ou du tout-trans-rétinaldéhyde, ou de l'acétate de tout-trans-rétinyle ou d'autres esters ou sels convenables de l'acide rétinoïque, pour l'utilisation défine dans la revendication 1.

3. Utilisation d'un rétinoïde choisi parmi les stéréo-isomères de l'acide vitamine A, de l'acide vitamine $A_2$, de l'acide γ-vitamine A, de l'acide α-vitamine A, de l'acide 5,6-époxy-vitamine A, de l'acide déhydro-vitamine A, ou des aldéhydes, alcools, esters, éthers ou sels dérivés des composés mentionnés ci-dessus, pour l'utilisation définie dans la revendication 1.

4. Utilisation d'un métabolite naturel de l'acide vitamine A, tel que l'acide 4-oxo-rétinoïque, l'acide 4-céto-rétinoïque, l'acide 4-oxo-rétinoïque, l'acide 5,8-oxy-rétinoïque ou le 7-cis-3-déhydrorétinol ou l'acide rétinoïque, l'acide 7-trans-9-cis-11-trans-13-trans-5'-hydroxy-rétinoïque, ou le 8-(2,6,6-triméthyl-3-oxocyclohex-1-ényl)-2,6-diméthyl-6-hydroxy-octatriénal, ou des stéréo-isomères des composés mentionnées ci-dessus, ou des aldéhydes ou d'autres dérivés comme les esters ou les sels de ceux-ci, pour l'utilisation définie dans la revendication 1.

5. Utilisation d'un caroténoïde, tel que la crocétine ou la crocine ou leurs métabolites naturels, pour l'utilisation définié dans la revendication 1.

6. Utilisation d'un rétinoïde, tel que défini dans l'une quelconque des revendications 1 à 5, pour la fabrication d'une composition pharmaceutique adaptée à l'administration locale pour le traitement de l'alopécie humaine par augmentation de la vitesse de croissance des cheveux et prolongement de la phase anagène du cycle du cheveu.

7. Utilisation d'un rétinoïde tel que défini dans l'une quelconque des revendications 1 à 5, dans laquelle le memmifère est un animal à fourrure et la composition agit également comme remède contre la mue ou la chute des poils.

8. Utilisation pour le propos défini dans les revendications 1 à 6, dans laquelle la composition pharmaceutique ou vétérinaire se présente comme une lotion, une crème, un onguent, un agent de conditionnement un shampooing, à application locale, et la concentration de rétinoïde se situe entre 0,001 et 5% en poids.

9. Utilisation telle que définie dans la revendication 8, dans laquelle la lotion à application locale contient, en tant que solubilisants, un mélange d'éthanol et de propylène-glycol.

10. Procédé de traitement des animaux à fourrure afin d'augmenter la vitesse de croissance de la fourrure, caractérisé en ce que l'on administre aux animaux une quantité efficace d'un rétinoïde tel que défini dans l'une quelconque des revendications 1 à 5.